# EUROPEAN PATENT APPLICATION

(11) **EP 3 372 194 A1**
(43) Date of publication of application: **12.09.2018**
(21) Application number: 16861641.5
(22) Date of filing: 02.11.2016
(51) Int. Cl.: A61C 13/30

(54) **INTRARADICULAR "ANCLA" POST**

(30) Priority: 05.11.2015 DO 2015000272
(71) Applicant: Feliz Matos, Leandro Edgardo, 22333 Gazcue, Distrito Nacional, Santo Domingo (DO); Mancebo Pacheco, Elvin Alberto, 22333 Gazcue, Distrito Nacional, Santo Domingo (DO); Pereyra Guerrero, Dulce Concepción, 22333 Gazcue, Distrito Nacional, Santo Domingo (DO); Universidad Iberoamericana, Unibe, 22333 Gazcue, Distrito Nacional, Santo Domingo (DO)
(72) Inventor: FELIZ MATOS, Leandro Edgardo, 22333 Gazcue, Distrito Nacional, Santo Domingo (DO); MANCEBO PACHECO, Elvin Alberto, 22333 Gazcue, Distrito Nacional, Santo Domingo (DO); PEREYRA GUERRERO, Dulce Concepción, 22333 Gazcue, Distrito Nacional, Santo Domingo (DO)
(74) Representative: Algemeen Octrooi- en Merkenbureau B.V.
(86) International application number: PCT/DO2016/000003
(87) International publication number: WO 2017/076417

(57) **Abstract**

The invention relates to an odontological biomaterial called "ANCLA Post". The post is used as a reconstruction means in anterior and posterior teeth, the cylindrical, conical design of the extension and anchor-shaped design of the apical end allowing forces to be adequately distributed to prevent the fracturing of the tooth and to keep same repairable and usable for a long time. The post is characterized in that it is made of fiberglass, resin or polymer, and is white and radiopaque.

## Description

Intraradicular ANCHOR pin for anterior and posterior teeth. The invention relates to an odontological biomaterial for the rehabilitation of anterior and posterior teeth treated endodontically, after a wide lesion of dental caries and with coronary destruction, serving as an intraradicular anchoring element and for supporting restorations.

Existing pins (posts) can be categorized into two large groups, metallic pins that can be cast and prefabricated and non-metallic pins based on fiberglass embedded in polymers, having as disadvantages the high incidence of irreparable dental fractures associated with them.

Both groups have been well studied via *in vitro* and *in vivo* clinical tests, so that the indications and contraindications of each one have been established in the literature. Reference is made to invention patents teaching similar devices. Patent #3199037(US4729736A) consists of a dental post to retain a dental restoration, formed by a cylindrical pin having a longitudinal axis, with helical grooves, also has an external contour (shelves) for helping retention inside the tooth. Patent #207971 which consists in a fastening device for dental prosthesis over a pin introduced in the dental root. Patent #RU2556526 consists of two components: pins and stumps. The pin component is made of computerized grinding ceramics and has an incrustation that can be accurately fixed to the walls of a prepared root canal Patent #USD730523 (S1), Dental Post (summary not available).

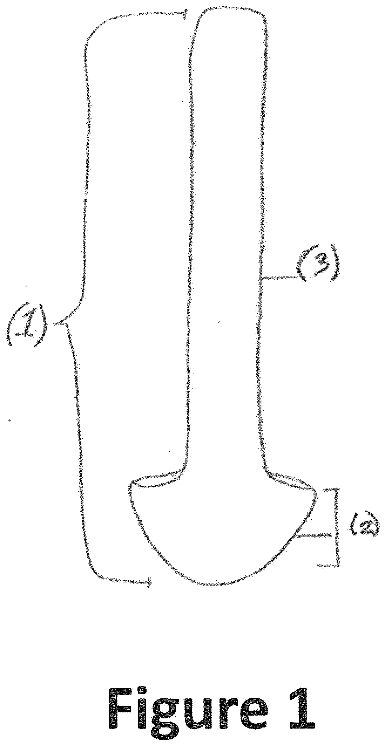

The ANCHOR pin, is an aesthetically prefabricated intraradicular retention device, which adapts to the apical end of the root canal once it has been unsealed passively residing within to allow reconstructing the stump with resinous materials to receive the final restoration, see Figure 1.

The total length of the intarradicular pin ranges from 5mm to 22mm from end to end, see Figure 1(1), with apical anatomical shape (ANCHOR), see Figure 1(2) according to the apical internal space of the dental root with a range of 1mm to 5mm long and diameter from 1.5mm to 7mm, see Figure 1(2).

The objective of the intraradicular ANCHOR pin is to allow the rehabilitation of all the dental organs (anterior and posterior teeth that need an intraradicular retention device to regain its main function of chewing as well as phonation, swallowing and aesthetic losses. In addition, it also allows the passive reconstruction of the tooth, decreases the tension in the lateral walls, improves the intraradicular element retention and offers the possibility of performing an aesthetic dental reconstruction. The ANCHOR pin requires little time for its installation in a tooth, and is inexpensive.

The ANCHOR pin is designed to comply with the typical static and dynamic functions of dental chewing under its indications, inserting and reconstructing techniques, resisting forces up to 1400 newton, tested in the MTI 2k universal assays system (USA). These characteristics allow that when there is a fracture in the ANCHOR pin, said fracture being of the removable type, and therefore the tooth can be repaired.

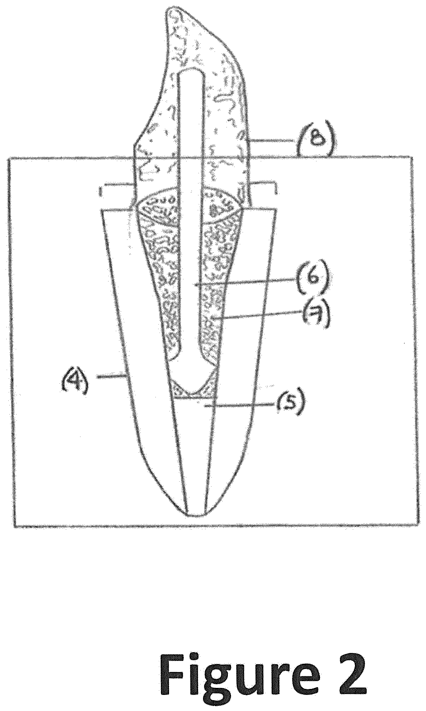

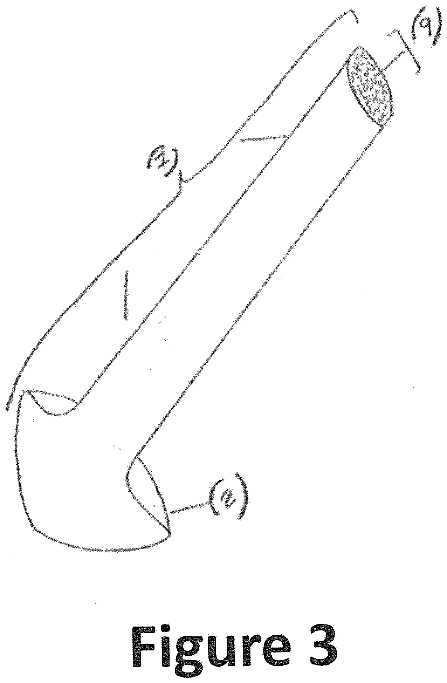

In order to use the ANCHOR pin a tooth root must be in good condition, and with endodontic treatment already performed (ducts), see Figure 2(1)(2). The material placed in the endodontics (gutta-percha) is removed leaving 5 mm of the apical seal or at the end of the root, see Figure 2(2).

The diameter and length of the ANCHOR pin to be used is selected with X-ray according to the root canal dimension, see Figure 2(3).

It is rectified with the drill-bit of the ANCHOR pin to be used avoiding circular movements, the intraradicular ANCHOR pin is tested and adjusted.

The duct is cleaned and further conditioning and placement of resinous cement of any brand inside the duct is carried out according to the cement manufacturer's recommendations, see Figure 2(4).

The ANCHOR pin is manually inserted slowly, the cement is allowed to set, normally by means of LED illumination and afterwards the bonding and hardness of the cement is verified, the stump is reconstructed (upper part of the tooth that supports the restoration with composite resin or resinous cement of any brand) and finally the stump is carved or prepared and the tooth is ready to receive a restoration which is usually a porcelain crown, see Figure 2(5).

## Claims

1. The ANCHOR is composed in its entirety of 30-80 percent fiberglass, and of 20-70 percent resin and polymers, see figure 1.

2. It is **characterized by** a translucent/white tone, see figure 1.

3. It has a straight and conical shape, see figure 3.

4. It is **characterized by** a cylindrical shape in its entirety until the ANCHOR, see figure 3(2).

5. Comprising an elastic flexing module in a range of 20-90GPA.

6. **Characterized by** a flexing force from 1000 to 1600 Mpa.

7. Comprising a compression force from 400 newton to 2000 newton.

8. It is characterized for being radiopaque in a range between 40% and 180%.

9. The ANCHOR pin is characterized for its classification according to its length in a range of 15 different sizes from 1 to 15 (from 5mm to 22mm), see figure 1(1), with a minimum diameter of 0.5mm and maximum of 3.0mm, see figure 3(2), with the ANCHOR in a minimum diameter of 0.7mm to 6mm, see figure 3(3).

10. These characteristics avoid the frequency of irreparable dental fracture, the ANCHOR pin being **characterized by** a fracture of the removable type where the tooth is totally restorable.

## Amended claims

### Amended claims under Art. 19.1 PCT

1. Intraradicular pin for anterior and posterior teeth **characterized by** a white translucid tone, and a cylindrical shape along its length until reaching the lower end where it ends in the form of an anchor improving retention of the intraradicular element.

2. Intraradicular pin for anterior and posterior teeth according to claim 1 **characterized by** having an elastic flexing modulus in a range of 20-90 Gpa, 30-80 percent of fiberglass and 20-70 percent of resin and polymers achieving a flexing force of 1000 to 1600 MPa, a compression force from 400 to 2000 Newton and being radiopaque in a range between 40 and 180 percent.

3. Intraradicular pin for anterior and posterior teeth according to claims 1 and 2, **characterized by** avoiding irreparable dental fracture and the total restoration of the tooth in its diameters from 07. To 6 mm and its length from 5 to 22 mm, being identified in numberings of 1-15.
